# EUROPEAN PATENT SPECIFICATION

(11) **EP 2 061 427 B1**
(45) Date of publication and mention of the grant of the patent: **20.07.2011**
(21) Application number: 07808579.2
(22) Date of filing: 14.09.2007
(51) Int. Cl.: A61K 9/00, A61K 9/16

(54) **GRANULATE CONTAINING A PHARMACEUTICALLY ACTIVE SUBSTANCE AND AN EMULSIFIER AND METHOD FOR ITS MANUFACTURE**
GRANULAT MIT EINEM PHARMAZEUTISCHEN WIRKSTOFF UND EINEM EMULGATOR UND HERSTELLUNGSVERFAHREN DAFÜR
GRANULAT MONOPHASIQUE COMPRENANT UN PRINCIPE ACTIF ET UN AGENT ÉMULSIFIANT, ET PROCÉDÉ POUR SA PRÉPARATION

(30) Priority: 15.09.2006 EP 06120741
(43) Date of publication of application: 27.05.2009
(73) Proprietor: Echo Pharmaceuticals B.V., 1382GS Weesp (NL)
(72) Inventor: PELLIKAAN, Hubert Clemens, 3572 TT Utrecht (NL); VERMEULEN, Pieter Sebastiaan, 3581 WT Utrecht (NL); BENDER, Johannes Caspar Mathias Elizabeth, 3581 XE Utrecht (NL); FERNANDEZ CID, Maria Vanesa, 2023 AE Haarlem (NL)
(74) Representative: Swinkels, Bart Willem
(86) International application number: PCT/NL2007/050448
(87) International publication number: WO 2008/033023

(56) References cited:
- EP-A1- 0 631 782
- EP-A1- 1 757 361
- WO-A-93/13757
- WO-A-2004/071645
- WO-A-2007/072106
- FR-A1- 2 758 459
- US-A1- 2005 008 691
- FRANCESCHINIS E ET AL: "Self-emulsifying pellets prepared by wet granulation in high-shear mixer: influence of formulation variables and preliminary study on the in vitro absorption" INTERNATIONAL JOURNAL OF PHARMACEUTICS, AMSTERDAM, NL, vol. 291, no. 1-2, 3 March 2005 (2005-03-03), pages 87-97, XP004737779 ISSN: 0378-5173
- CHAMBIN O ET AL: "INTEREST OF MULTIFUNCTIONAL LIPID EXCIPIENTS: CASE OF GELUCIRE 44/14" DRUG DEVELOPMENT AND INDUSTRIAL PHARMACY, NEW YORK, NY, US, vol. 31, no. 6, July 2005 (2005-07), pages 527-534, XP009059038 ISSN: 0363-9045

## Description

### TECHNICAL FIELD OF THE INVENTION

The present invention relates to a granulate containing a pharmaceutically active substance and an emulsifier. The granulates of the present invention are particularly suited for use in dosage units that are intended for transmucosal (e.g buccal, sublingual, peroral, nasal, pulmonary, rectal, intrauterine or intravaginal) administration.

The invention also provides a method for the manufacture of such a granulate.

### BACKGROUND OF THE INVENTION

In order for pharmaceutically active substances to be able to deliver a systemic pharmaceutical effect, following administration such pharmaceutically active substances have to be absorbed into the bloodstream. In particular if pharmaceutical substances are administered transmucosally, it is important that the pharmaceutically active substance is rapidly released from a pharmaceutical dosage unit into the aqueous environment found in the vicinity of mucosal tissue so that it can be absorbed by said mucosal tissue. Especially in case a pharmaceutically active substance is essentially water-insoluble or poorly water-dispersible, it poses a major challenge to formulate a delivery system that will achieve a fast release of the pharmaceutically active substance in the musosal fluid in a way that enables effective absorption of the pharmaceutically active substance by the mucosal tissue.

US 4,840,799 describes a process for the preparation of a rapidly disintegrating core comprising a pharmaceutically active compound, said process comprising adding an emulsifier/tenside to a solution of the pharmaceutically active compound in a water:alcohol mixture to form a pumpable composition, subjecting the pumpable composition to an intense treatment in an apparatus selected from the group homogeniser, colloid mill, pearl mill, and pebble mill wherein the composition is distributed over a bed of at least one solid material having a large effective surface area and having nonbinding properties to the said pharmaceutically active compound so as to form an agglomerate, and drying the agglomerate to form a granulate having irregular or spherical form.

WO 97/36577 describes a dry solid lipid composition comprising: a first component of a lipophilic substance in an amount sufficient to provide a therapeutic effect when administered to a mammal; a second component of a lipid comprising at least one solid fat; and a third component of at least one phospholipids, wherein the second and third components are present in an amount sufficient to increase the bioavailability of the lipophilic substance when administered to the mammal. Lipophilic substances are said to include drugs. Cannabinoids, hormones and vitamins are mentioned as examples of lipophilic substances. The dry solid lipid compositions described in WO 97/365577 are said to be useful for the oral delivery of lipophilic substances. More particularly, it is said that the dry solid lipid compositions have shown unexpectedly high drug-loading efficiency and enhanced oral bioavailability for the lipophilic compounds.

WO 97/36577 also describes a method for producing the aforementioned dry solid lipid composition, said method comprising:
- dissolving the three components in a suitable organic solvent;
- evaporating the solvent to complete dryness;
- hydrating the resulting dry solid lipid mixture with an aqueous phase to obtain a lipid dispersion in water;
- homogenizing the resultant lipid dispersion to reduce the particle size to the submicron range; and
- drying the homogenized dispersion to form the lipid dry-drug mixture.

US 4,719,239 describes a pharmaceutical multicomponent system for transdermal (systemic) use of lipophilic active agents, comprising:
- 0.1-15 wt.% of solid active agent or up to 65 wt.% of liquid active agent;
- 1-50 wt.% of surfactant;
- 0-80 wt.% of cosurfactant; and
- 0-85 wt.% of oil component,
   the sum of the cosurfactant and oil components being at least 2.5 wt.%. Granulates are nowhere mentioned.

US 5,342,625 describes pharmaceutical compositions comprising a cyclosporine, a hydrophilic phase component, a lipophilic phase component and a surfactant, wherein said composition is a microemulsion preconcentrate, which upon dilution with water is capable of providing an oil-in-waler microemulsion. It is stated in US 5,342,625 that suitable dosage forms for oral administration include e.g. liquids, granulates and the like. The preferred dosage forms are unit dosage forms, for examples tabletted or encapsulated forms, in particular hard or soft gelatine encapsulated forms.

US 6,054,136 describes a substantially anhydrous pharmaceutical composition for oral administration that is capable of forming a microemulsion *in situ* with the biological fluid of the body. This pharmaceutical composition comprises:
i. a lipophilic phase consisting of a mixture of fatty acid esters and glycerides;
ii. a surfactant with an HLB of less than 16 that is chosen from the group comprising polyglycolysed glycerides and oleic esters of polyglycerol;
iii. a cosurfactant chosen from the group comprising lauric esters of propylene glycol, oleic esters of polyglycerol and ethyl diglycol; and
iv. a pharmaceutically active ingredient.

In the US patent it is observed that the pharmaceutical composition described therein forms a microemulsion in the presence of the physiological fluid of the stomach and intestine of the human or animal body without the need of supplying an external hydrophilic phase to produce this microemulsion. Granulates are nowhere mentioned.

WO 2007/024133, published after the priority date of the present patent application, describes a process for the preparation of encapsulates, which process employs:
- a pumpable emulsion comprising (i) a continuous phase containing a solvent and a matrix-forming solute dissolved in said solvent and (ii) a dispersed phase;
- an extractant comprising supercritical, subcritical or liquefied gas; said solvent being substantially more soluble in the extractant than said matrix-forming solute and said process comprising the successive steps of:
   a. combining the pumpable emulsion with the extractant under mixing conditions;
   b. allowing the formation of particulate encapsulates in which the dispersed phase is encased in a solid matrix of the matrix-forming solute;
   c. collecting the encapsulates and separating them from the extractant.

Example 2 of this international patent application describes the preparation of an encapsulate from an emulsion containing 11.15 g inulinc, 30 ml water, 1.62 g whey powder, 1.0 g Tween-20 and 5.98 g molten cannabis. This emulsion was sprayed together with CO₂ via a two-fluid nozzle into a pressurised high pressure vessel (6 litre). The vessel was heated via a jacket to 40°C and pressurized to 30 bar. A dry encapsulate was thus obtained.

### SUMMARY OF THE INVENTION

The inventors have developed a granulate that contains at least 0.1 wt.% of a pharmaceutically active substance and that can advantageously be used in the manufacture of pharmaceutical dosage units for transmucosal delivery. In particular, the granulates of the present invention enable the manufacture of transmucosal dosage units that exhibit improved solubility and/or bioavailability of the pharmaceutically active substance.

The granulates of the present invention are further characterised in that they have a volume weighted mean diameter of 1-200 µm, in that they contain a substantial amount, i.e. a least 10 wt.% of emulsifier selected from the group consisting of sugar fatty acid esters, mono-glycerides, di-glycerides, diacetyl tartaric acid ester of monoglyceride, diacetyl tartaric acid esters of diglyceride, polyglycerol esters, calcium stearoyl lactylate, sodium stearoyl lactylate and combinations thereof; and 0-89.9 wt.% of a water-dispersible saccharide, that the combination of pharmaceutically active substance, the emulsifier and the water-dispersible saccharide together representing at least 60 wt.% of the granulate; wherein the granulate is monophasic or wherein the granulate comprises a dispersed phase containing the pharmaceutically active substance, said dispersed phase having a volume weighted mean diameter of less than 300 nm.

The granulates of the present invention offer the advantage that, following administration, the pharmaceutically active substance is rapidly and essentially completely released in the biological fluid found near mucosal tissue even if said active substance *per se* is essentially water-insoluble or poorly water-dispersible. Although the inventors do not wish to be bound by theory, it is believed that the combination of the particulate nature of the granulates, the relatively small diameter of the granules and the high emulsifier content greatly facilitates the dispersal of the pharmaceutically active substance into the biological fluid. In addition, if the pharmaceutically active substance is essentially water-insoluble, the emulsifier stimulates the formation of a very fine (micro-)emulsion of the substance in the biological fluid, thus enabling fast absorption of said substance by the mucosal tissue.

Besides aiding the delivery of the pharmaceutically active substance, the emulsifier also serves the purpose of protecting the pharmaceutically active substance against degradation under the influence of e.g. oxygen, UV-light, reactive substances and/or moisture.

The inventors have also developed a process for the preparation of a granulate containing a pharmaceutically active substance, which process employs:
- a pumpable emulsion comprising (i) a continuous phase containing at least 30 wt.% of a polar solvent and (ii) a dispersed phase containing at least 10 wt.% of an emulsifier and at least 0.1 wt.% of a pharmaceutically active substance;
- an extractant comprising at least 60 wt.% of a supercritical, subcritical or liquefied gas;
   said solvent being substantially more soluble in the extractant than said emulsifier; the process comprising the successive steps of :
   a. combining the pumpable emulsion with the extractant under mixing conditions;
   b. allowing the formation of granules containing the emulsifier and the pharmaceutically active substance;
   c. collecting the granules and separating them from the extractant.

### DEFINITIONS

The term "granulate" as used herein refers to a particulate material that consists of discrete particles. The discrete particles within the granulate of the present invention contain both the emulsifier and the pharmaceutically active substance.

The term "monophasic" as used herein refers to a granulate that essentially consists of a single lipophilic phase. In the single lipophilic phase the pharmaceutically active substance and the emulsifier can be present in the form of a (solid) solution or in the form of a molecular dispersion. Thus a monophasic granulate of the present invention does not contain two or more immiscible phases, e.g. a lipophilic and a hydrophilic phase. A "molecular dispersion" is a dispersion in which the dispersed phase consists of individual molecules. If the molecules are of less than colloidal size, the result is a true solution.

The term "emulsifier" as used herein refers to a surface active component comprising one or more substances having a polar or ionic portion and a non-polar, e.g. aliphatic portion, which surface active component is capable of stabilising an emulsion, especially an oil-in-water emulsion. It is noted that the present invention encompasses the use of an emulsifier containing two or more surface active substances, notably a combination of an O/W emulsifier and a co-emulsifier.

The term "O/W emulsifier" as used herein refers to a surface active component that facilitate oil-in-water emulsification. Typically, O/W emulsifiers exhibit an HLB-value of 3-7.

The term "co-emulsifier" as used herein refers to a surface active component with hydrophobic character that is capable of enhancing the oil-in-water emulsification properties of an O/W emulsifier with which it is combined. Typically, co-emulsiliers exhibit an HLB value of at least 8.

The term "saccharide" as used herein encompasses monosaccharides, disaccharides, oligosaccharides comprising 3-10 sugar components and polysaccharides comprising at least 11 sugar components.

The term "transmucosal administration" as used herein refers to a mode of administration in which a dosage unit containing the pharmaceutically active substance is positioned or delivered near a mucosal tissue and wherein the pharmaceutically active substance exerts its pharmaceutical effect after having been absorbed by said mucosal tissue. Examples of transmucosal administration include: buccal, sublingual, peroral, nasal, pulmonary, rectal, intrauterine and intravaginal administration.

The term "transmucosal dosage unit" refers to a dosage unit that is designed for and suitable for transmucosal administration of a pharmaceutically active substance contained therein.

The term "oral" as used herein, unless indicated otherwise, refers to a mode of administration that involves insertion of a pharmaceutical dosage unit into or through the mounth. Examples of oral administration include peroral, buccal and sublingual administration.

The term "peroral" as used herein refers to a mode of administration that involves ingestion of the dosage unit without significant residence time in the oral cavity.

The term "subcritical gas" as used herein refers to a compressed gas that is neither in a supercritical or liquefïed state but that has been pressurised to at least 10 bar, preferably to at least 20 bar.

### DETAILED DESCRIPTION OF THE INVENTION

One aspect of the present invention relates to a granulate having a volume weighted mean diameter of 1-200 µm and containing:
- at least 0.1 wt.% of a pharmaceutically active substance;
- at least 10 wt.% of emulsifier selected from the group consisting of sugar fatty acid esters, mono-glycerides, di-glycerides, diacetyl tartaric acid ester of monoglyceride, diacetyl tartaric acid esters of diglyceride, polyglycerol esters, calcium stearoyl lactylate, sodium stearoyl lactylate and combinations thereof; and
- 0-89.9 wt.% of a water-dispersible saccharide;
- the combination of the pharmaceutically active substance, the emulsifier and the water-dispersible saccharide together represening at least 60 wt.% of the granulate;
   wherein the granulate is monophasic or wherein the granulate comprises a dispersed phase containing the pharmaceutically active substance, said dispersed phase having a volume weighted mean diameter of less than 300 nm, preferably of less than 100 nm.

According to a particularly preferred embodiment, the present granulate is a free flowing powder. Preferably, the present granulate has a volume weighted mean diameter of at least 3 µm, more preferably of at least 5 µm. The volume weighted mean diameter of the granulate preferably does not exceed 100 µm, more preferably it does not exceed 80 µm. The volume weighted mean diameter of the granulate may suitably be determined by means of image analysis methods.

Besides the pharmaceutically active substance and the emulsifier, the present granulate may suitably contain other components such as anti-oxidants, preservatives, fat, wax, further pharmaceutically active substances, etc.

According to one embodiment of the present invention, the present granulate is a monophasic granulate. As explained herein before the monophasic granulate of the present invention may take the form of a solution or a molecular dispersion. Most preferably, the monophasic granulate consists a single lipophilic phase in the form of a (true) solution. In the monophasic granulate or the present invention the pharmaceutically active substance and the emulsifier together preferably represent at least 60 wt.% of the granulate. According to a particularly preferred embodiment, the pharmaceutically active substances and the emulsifier together represent at least 80 wt.% of the present granulate.

The present invention encompasses the use of a large variety of emulsifiers. Preferably, the emulsifier employed in according to the present invention comprises a non-ionic emulsifier.

According to another preferred embodiment, the emulsifier comprises an ester containing 1 to 4 C₆₋₂₄ fatty acid residues. These fatty acid residues are comprised in the lipophilic part of the emulsifier.

The emulsifier employed in the present granulate advantageously contains at least one free hydroxyl group, preferably at least two free hydroxyl groups. The free hydroxyl groups are comprised in the hydrophilic part of the emulsifier.

The hydrophilicity and lipophilicity are different among emulsifiers, and the balance between the two is called HLB value. HLB-values can range from 0 to 20. An emulsifier with higher lipophilicity shows a lower HLB whereas higher hydrophilicity has is reflected in a higher HLB. According to a particularly, preferred embodiment, the emulsifier comprises an O/W emulsifier with an HLB-value of more than 6, more preferably 8-18. In addition the aforementioned O/W emulsifier, the emulsifier may suitably comprise a co-emulsifier, especially a co-emulsifier with an HLB of not more than 6.

According to a particularly preferred embodiment, the emulsifier employed in accordance with the present invention is a sugar fatty acid ester, more preferably a sugar fatty acid ester containing 1-3 fatty acid residues per molecule. Even more preferably the sugar fatty acid ester contains 1 or 2 acid residues per molecule. Most preferably, the sugar fatty acid ester contains one fatty acid residue per molecule. The sugar residue in the aforementioned sugar fatty acid esters most preferably is a sucrose residue.

The amount of pharmaceutically active substance contained in the present granulate typically is within the range of 0.1-50 wt.%. Preferably, the granulate contains 0.3-30 wt.% of the pharmaceutically active substance. The advantageous properties of the present granulate arc most pronounced in case the pharmaceutically active substance is essentially completely dissolved in the granulate matrix. Thus, in a preferred embodiment, the granulate contains from 0.1-50%, even more preferably 0.3-30 wt.% of dissolved pharmaceutically active substance.

As explained herein before, the granulates of the present invention are particularly suited for delivering pharmaceutically active substances that are essentially water-insoluble or poorly dispersible in water. Accordingly, in a preferred embodiment, the pharmaceutically active substance is a lipophilic or an amphipathic substance. Most preferably, the pharmaceutically active substance is a lipophilic substance. Typically, water solubility of the pharmaceutically active substance at 35 °C is less than 1 mg/ml. Preferably, water solubility at 35 °C is less than 0.1 mg/ml

Examples of pharmaceutically active substances that may advantageously be incorporated in the present granulate include cannabinoids, steroids, flavonoids, polyphenols and combinations thereof. Preferred steroids are terpenoid lipids that comprises a carbon skeleton with four fused rings, arranged in a 6-6-6-5 fashion.

The incorporation of a water-dispersible saccharide in the present granulate may aid the formation of a micro-emulsion, especially if the emulsifier and pharmaceutically active substance are finely dispersed throughout a matrix of said water dispersible saccharide. The optional water-dispersible saccharide in the present granulate advantageously has a solubility in water of 35 °C of at least 10 mg/ml, preferably of at least 30 mg/ml. Furthermore, the water-dispersible saccharide preferably has a molecular weight of less than 10,000 preferably of less than 6,000 g.

Examples of saccharides that may be employed in the present granulate include maltodextrin, trehalose, cellobiose, glucose, fructose, maltulose, iso-maltulose, lactulose, maltose, gentobiose, lactose, isomaltose, maltitol, lactitol, erythritol, palatinitol, xylitol, mannitol, sorbitol, dulcitol, ribitol, sucrose, raffinose, gentianose, planteose, verbascose, stachyose, melezitose, dextran, and inositol.

In accordance with an advantageous embodiment of the present invention, the granulate is capable of forming a microemulsion when said granulate is dispersed in an adequate quantity of water having a temperature of 37 °C. Microemulsions arc identifiable as possessing one or more, preferably all of the following characteristics:
- They are formed spontaneously or essentially unforced when their components are brought into contact, that is without substantial energy supply, e.g. in the absence of heating or the use of high shear equipment or other substantial agitation.
- They exhibit thermodynamic stability.
- They are substantially non-opaque, i.e. are transparent or opalescent when viewed by optical microscopic means.
- Microemulsions comprise a dispersed or particulate (droplet) phase, the particles of which are of a size less than 2,000 A°.

The present invention also provides a process for the preparation of the present granulate that offers the advantageous that it enables the preparation of a granulate with a very homogeneous particle size distribution. A homogeneous particle size distribution is desirable as non-homogeneous granulates show a tendency to segregate. The granulate of the present invention advantageously exhibits a homogeneous particle size distribution as evidenced by the fact that the ratio of the standard deviation to the average diameter is less than 100%.

The present invention also provides a solid or semi-solid pharmaceutical dosage unit comprising at least 10 wt.%, preferably at least 20 wt.% of the granulate according to the present invention. Besides the granulate, the pharmaceutical dosage unit may suitably contain up to 90 wt.% of excipient, preferably a water-soluble excipient. Examples of pharmaceutical dosage units encompassed by the present invention are tablets, capsules, granulates, suppositories and gels. Most preferably, the dosage unit is a tablet for oral administration, especially sublingual or buccal administration.

Another aspect of the invention relates to the use of the present granulate in the manufacture of a pharmaceutical dosage unit, said manufacture comprising the incorporation of the granulate in the pharmaceutical dosage unit.

A further aspect of the invention relates to the use of a pharmaceutical dosage unit containing the present granulate in a method of therapeutic or prophylactic treatment, said treatment comprising transmucosal administration of the dosage unit. As explained herein before examples of transmucosal administration include buccal, sublingual, peroral, nasal, pulmonary, rectal, intrauterine and intravaginal administration. Preferably, the treatment comprises oral (e.g. peroral, buccal or sublingual) or pulmonary administration, even more preferably oral administration. Most preferably, the treatment comprises sublingual or buccal administration of the pharmaceutically active substance. The pharmaceutical dosage unit of the present invention is advantageously employed in the treatment of mammals, most preferably of humans.

Yet another aspect of the present invention relates to a process for the preparation of a granulate containing a pharmaceutically active substance, which process employs:
- a pumpable emulsion comprising (i) a continuous phase containing at least 30 wt.%, preferably at least 50 wt.% of a polar solvent and (ii) a dispersed phase containing at least 10 wt.% of an emulsifier and at least 0.1 wt.% of a pharmaceutically active substance;
- an extractant comprising at least 60 wt.% of a supercritical, subcritical or liquefied gas;
   said solvent being substantially more soluble in the extractant than said emulsifier;
   the process comprising the successive steps of :
   a. combining the pumpable emulsion with the extractant under mixing conditions;
   b. allowing the formation of granules containing the emulsifier and the pharmaceutically active substance;
   c. collecting the granules and separating them from the extractant.

According to a preferred embodiment, the pumpable emulsion and the extractant are combined by feeding a stream of the pumpable emulsion and a stream of the extractant into a mixing zone where both streams are thoroughly mixed. The combined streams are advantageously transferred from the mixing zone into another zone (the precipitation zone), in which granule formation is allowed to take place under controlled conditions. The present method offers the advantage that it can suitably be operated in a continuous or semi-continuous fashion.

The pumpable emulsion of the present process is advantageously combined with the extractant by spraying the emulsion into a mixing zone containing the extractant by means of a nozzle with an inner diameter of more than 1 mm. The use of a nozzle with a relatively large diameter offers the advantage that the emulsion is not broken upon introduction into the mixing zone.

In accordance with a preferred embodiment, the emulsion and the extractant are admixed in a weight ratio within the range of 1:5 to 1:500, preferably of 1:10 to 1:300.

The pumpable emulsion or the present process is suitably formed by combining the solvent with the one or more pharmaceutically active substances and/or emulsilier that are to constitute the dispersed phase, accompanied by or followed by homogenisation. Preferably, the emulsifier is combined with the solvent when said emulsifier is in a liquid (e.g. molten) state.

The pumpable emulsion employed in the present process suitably contains between 5 and 60 wt.%, preferably between 30 and 50 wt.% of the dispersed phase. The dispersed phase of the pumpable emulsion preferably has a volume weighted average droplet size of 1-200µm, more preferably of 3-100 µm.

In principle, any combinations of emulsifier, solvent and extractant may be employed in the present method, provided the solvent is soluble in the extractant and the emulsifier is substantially less soluble in the extractant. Preferably, under the extraction conditions employed in the process, the solubility of the solvent in the extractant exceeds 0.1% (w/w), preferably exceeds 0.5% (w/w).

Generally, solubility of the emulsifier in the extractant does not exceed 5% (w/w). Preferably, under the extraction conditions employed in the present process solubility of the emulsifier in the extractant is at least 100, more preferably at least 1000 times lower than the solubility of the solvent in the same extractant.

The polar solvent is advantageously selected from the group consisting of: C₁₋₆ alchohols, ketones, water and combinations thereof. Even more preferably, the organic polar solvent is selected from the group consisting of: water, methanol, ethanol, propanol, iso-propanol, acetone, and mixtures thereof.

The inventors have unexpectedly found that excellent results can be obtained with the present method even if the dispersed phase is equally or even more soluble in the extractant than in the solvent. Although the inventors do not wish to be bound by theory, it is believed that the solubility of the dispersed phase in the solvent is conversely correlated with the efficacy of the present method. Accordingly, the solubility of the dispersed phase in the solvent preferably does not exceed 0.1 wt.%, more preferably it does not exceed 0.01 wt.%. In case the dispersed phase comprises an emulsion, the continuous phase of said emulsion should meet the aforementioned solubility criteria.

The supercritical subcritical or liquefied gas employed in the present process is preferably selected from the group consisting of carbon dioxide, nitrous oxide, ethane, ethylene propane, cyclopropane, propylene, butane, argon, nitrogen and mixtures thereof. Most preferably, said gas comprises supercritical, subcritical or liquefied carbon dioxide.

In the present process contact time between extractant and the precipitated granules is preferably kept as short as possible in order to prevent that emulsifier and/or pharmaceutically active substance are extracted from the encapsulates. Typically, average contact time between granules and extractant does not exceed 60 minutes. Preferably, the contact time does not exceed 30 minutes, most preferably it does not exceed 10 minutes.

According to a particularly preferred embodiment, the granules are separated from the extractant whilst precipitation continues. This may be achieved, for instance, with the help of a cyclone or by collecting the particles in a medium that is immiscible with extractant.

In a preferred embodiment of the present process, following separation of the extractant, the extracted solvent is removed from the extractant and the extractant is recirculated to step a. of the process. Thus, the total amount of extractant employed in the process may be minimised without significant adverse effects on process efficiency or encapsulate quality. In a particularly preferred embodiment, the extracted solvent is effectively removed in a highly selective fashion. By removing the solvent and not, for instance, dissolved components of the dispersed phase, undesired extraction of the dispersed phase from the encapsulates may be avoided. Advantageously, the solvent is removed with an efficiency that is at least 10 times, preferably at least 100 times higher than the removal of dispersed phase components.

Solvent may be removed effectively from the extractant by employing an adsorbent or absorbent that adsorbs/absorbs the solvent but not the extractant. Alternatively, solvent is removed by reducing the pressure or temperature of the solvent-containing extractant to allow the solvent to condense. It is also feasible to remove the solvent by using selective membranes. Following separation of the extractant from the condensed solvent, the extractant is repressurised before being recirculated to step a.

In a particular embodiment of the invention the polar solvent contains water and the extracted water is removed from the extractant by contacting the extractant with a water adsorbent or a water absorbent that is insoluble in said extractant.

In the present process the extractant, when it is combined with the pumpable emulsion, preferably has a pressure of at least 10 bar, even more preferably of at least 20 bar. According to a particularly preferred embodiment, the extractant is a liquefied or supercritical gas having a pressure of at least 0.3xP_{c} and a temperature of at least T_{c}-60 °C, P_{c} representing the critical pressure of the gas and T_{c} representing the critical temperature of the gas.

The separated granules obtained from the present process typically have a volume weighted average diameter of 1-200 µm, more preferably of 3-100 µm, most preferably of 5-80 µm.

The invention is further illustrated by the following examples.

### EXAMPLES

### Example 1

Sucrose monolaurate (HLB=15) and tetrahydrocannabinol (THC) were heated under a stream of nitrogen till 120°C. The THC to sucrose monolaurate ratio was 1:15 by weight. After thoroughly mixing, the putty-like melt was saturated with CO₂ (and thereby softened) following one of either method below:
- The warm melt was poured into a 120°C-preheated autoclave and brought to 250 bars. The autoclave was pressurized with carbon dioxide using a plunger pump (LeWa) and heated by means of a jacket, using heating oil. The lump was further liquefied through saturation with CO₂ by stirring the melt in the supercritical CO₂ for al least 30 mins using a Büchi™ magnetic stirrer.
- The melt was chilled to -20°C and crushed to obtain maximum surface area. To this end a -20°C pre-cooled mortar was used in an inert and dry atmosphere. The obtained powder was poured into a 60°C pre-warmed autoclave and brought to 250 bars. The autoclave was pressurized with carbon dioxide using a plunger pump (LeWa) and heated by means of a jacket, using heating oil. The vessel was further heated to 120°C with heating oil and hot CO₂ (120°C) under continuous stirring allowing optimal CO₂-dissolvation.

After germinating the stirring, the melt was allowed to settle at the bottom of the autoclave. The valve at the bottom of the autoclave was opened. The high pressure in the autoclave forced the melt through a 120°C-heat traced pipe into a 120°C-heat traced 340 µm nozzle (Spraying Systems Inc). Powder was formed upon depressurization from 250 bars to atmospheric pressure. The microgranulate had an average diameter of 30 µm as determined by light microscopy.

### Example 2

THC was dissolved into a solution of 65% (w/v) sucrose monolaurate in 2-propanol to a finial concentration of 7.3% (w/v). A high pressure vessel was pressurized to 200 bars with carbon dioxide using a plunger pump (Williams) and heated to 40°C by means of a jacket, using heating oil.

The solution was sprayed into the vessel by means of a syringe pump (ISCO 260D) via a co-axial nozzle. This nozzle consists of two concentric tubes; 25 ml/min of the emulsion was fed via the inner tube (internal diameter = 0.5 mm, external diameter = 1.27 mm) and 500 g/min of carbon dioxide was fed via the outer tube (internal diameter = 1.7 mm). The carbon dioxide was heated to 60°C before being sprayed. The powder that formed within the vessel was collected on a filter at the bottom of the vessel. The microgranulates had an average diameter of 5-50 µm as determined by S.E.M.

### Example 3

Water was mixed with sucrose stearate (Crodesta™ F-160; HLB=15). The mixture was warmed till 60°C and melted THC was dispersed into this mixture, using an Ultraturrax™ stirrer, until a stable emulsion had formed. The resulting oil-in-water emulsion contained 10 wt.% of THC, 30 wt.% sucrose stearate and 60 wt.% of water. An autoclave was heated to 40°C by means of a jacket, using heating oil.

The autoclave was brought to 30 bars with CO₂ and the solution was sprayed into the vessel by means of a syringe pump (ISCO 260D) through a two fluid nozzle. This nozzle consists of two concentric tubes; 0.3 ml/min of the emulsion was fed via the inner tube (internal diameter = 0.5 mm, external diameter = 1.27 mm) and 500 g/min of carbon dioxide via the outer tube (internal diameter = 1.7 mm). The carbon dioxide was heated to 40°C before being sprayed. The powder that formed within the vessel was collected on a filter at the bottom or the vessel. The microgranulates had an average diameter or 5-50 µm as determined by S.E.M. By varying flow rates and the nozzle parameters the particle size can be varied within a wide range.

### Example 4

Water was mixed with sucrose stearate (Crodesta™ F-160; HLB=15) and glyceryl monostearate (sucrose stearate : glyceryl monostearate is 10:1 [w/w]). The mixture was warmed till 60°C and melted THC was dispersed into this mixture, using an Ultraturrax™ stirrer, until a stable emulsion had formed. The resulting oil-in-water emulsion contained 10 wt.% of THC, 30 wt% emulsifiers and 60 wt.% of water. An autoclave was heated to 40°C by means of a jacket, using heating oil.

The autoclave was brought to 30 bars with CO₂ and the solution was sprayed into the vessel by means of a syringe pump (ISCO 260D) through a two fluid nozzle. This nozzle consists of two concentric tubes; 0.5 ml/min of the emulsion was fed via the inner tube (internal diameter = 0.5 mm, external diameter = 1.27 mm) and 500 g/min of carbon dioxide via the outer tube (internal diameter = 1.7 mm). The carbon dioxide was heated to 40°C before being sprayed. The powder that formed within the vessel was collected on a filter at the bottom of the vessel. The microgranulates had an average diameter of 5-50 µm as determined by S.E.M.

### Example 5

A microgranulate containing tetrahydrocannabinol (THC) was prepared as follows.

An emulsion was prepared containing sucrose monolaurate (HLB=15), threhalose, THC, vitamin C and vitamin E (weight ratio: 4:20:1:0.05:0.05) in a concentration of 25 % (w/w) in DMSO

The emulsion was transferred to a syringe pump (ISCO). The emulsion was then pumped at a flow rate of 5 ml/min and sprayed via a co-axial nozzle to the vessel against a flow of CO₂ at 600 g/min. The vessel was previously pre-heated at 40 °C and pressurized with supercritical CO₂ at 100 bar. The carbon dioxide was also heated to 40 °C before being sprayed. The microgranulate that formed within the vessel was collected in a filter at the bottom of the vessel. The microgranulate had a mass weighted average diameter of 50 µm.

### Example 6

A microgranulate containing tetrahydrocannabinol (THC) was prepared as follows.

An emulsion was prepared containing sucrose monolaurate (HLB=15), maltodextrine 20, THC, vitamin C and vitamin E (weight ratio: 4:20:1:0.05:0.05) in a concentration of 25 % (w/w) in DMSO.

The emulsion was transferred to a syringe pump (ISCO). The emulsion was then pumped at a flow rate of 1.5 ml/ and sprayed via a co-axial nozzle to the vessel together with a flow of CO₂ at 600 g/min. The vessel was previously pre-heated at 40 °C and pressurized with supercritical CO₂ at 90 bar. The carbon dioxide was also heated to 40 °C before being sprayed. The microgranulate that formed within the vessel was collected in a filter at the bottom of the vessel. The microgranulate had a mass weighted average diameter of 50 µm.

### Example 7

A tabletting powder for direct compression was mixed using the following ingredients:
- 50 mg of the microgranulate described in Example 1
- 4 mg SiO₂ (aerosil)
- 15 mg sodium starch glycolate (Primojel™)
- 60 mg NaHCO₃
- 50 mg citric acid (1 aq.)

The powder was compressed applying a 15kN force to obtain a 10mm tablet with a total weight of 129 mg. The tablet strength was 40N and the tablet disintegrated in 60 seconds in water of 37°C, forming a micro emulsion.

The powder mixing and tabletting was performed in a dry and inert atmosphere.

### Example 8

A tabletting powder for direct compression was mixed using the following ingredients:
- 5g of the microgranulate described in Example 1
- 10 g maltodextrin
- 5g lactose
- 2g sodium starch glycolate (Primojel™)
- 0.05g aerosil
- 0.05g magnesium stearate.

The powder was compressed applying a 15kN force to obtain 7mm tablets with a total weight of 60mg. The tablet strength was 25N and the tablet disintegrated in 5 minutes in water of 37°C, forming a micro emulsion.

### Example 9

A tabletting powder for direct compression was mixed using the following ingredients:
- 5g of the microgranulate described in Example 1
- 15 g sorbitol
- 0.2g magnesium stearate.

The powder was compressed applying a 15kN force to obtain 7mm tablets with a total weight of 60mg. The tablet strength was 40N and the tablet disintegrated in 4½ minutes in water of 37°C, forming a micro emulsion.

## Claims

1. A granulate having a volume weighted mean diameter of 1-200 µm and containing:
• at least 0.1 wt.% of a pharmaceutically active substance;
• at least 10 wt.% of emulsifier selected from the group consisting of sugar fatty acid esters, mono-glycerides, di-glycerides, diacetyl tartaric acid ester of monoglyceride, diacetyl tartaric acid esters of diglyceride, polyglycerol esters, calcium stearoyl lactylate, sodium stearoyl lactylate and combinations thereof; and
• 0-89.9 wt.% of a water-dispersible saccharide;
• the combination of the pharmaceutically active substance, the emulsifier and the water-dispersible saccharide together represening at least 60 wt.% of the granulate;
wherein the granulate is monophasic or wherein the granulate comprises a dispersed phase containing the pharmaceutically active substance, said dispersed phase having a volume weighted mean diameter of less than 300 nm.

2. Granulate according to claim 1, wherein the emulsifier has a HLB-value of more than 6, preferably of 8-18.

3. Granulate according to claim 1 or 2, wherein the emulsifier is a sugar fatty acid ester.

4. Granulate according to any one of the preceding claims, wherein the pharmaceutically active substance is selected from the group consisting of cannabinoids, steroids, flavonoids, polyphenols and combinations thereof.

5. Granulate according to any one of the preceding claims, wherein the water-dispersible saccharide has a molecular weight of less than 10,000 preferably of less than 6,000 g.

6. Solid or semi-solid pharmaceutical dosage unit comprising at least 10 wt.%, preferably at least 20 wt.% of the granulate according to any one of the preceding claims.

7. Use of a granulate according to any one of claims 1-5 in the manufacture of a pharmaceutical dosage unit.

8. Use according to claim 7, wherein the pharmaceutical dosage unit is for use in a method of therapeutic or prophylactic treatment, said treatment comprising transmucosal administration of the pharmaceutical dosage unit.

9. Use according to claim 8, wherein the treatment comprises sublingual, buccal, pulmonary or peroral administration of the pharmaceutical dosage unit.

10. A process for the preparation of a granulate containing a pharmaceutically active substance, which process employs:
• a pumpable emulsion comprising (i) a continuous phase containing at least 30 wt.% of a polar solvent and (ii) a dispersed phase containing at least 10 wt.% of an emulsifier and at least 0.1 wt.% of a pharmaceutically active substance;
• an extractant comprising at least 60 wt.% of a supercritical, subcritical or liquefied gas;
said solvent being substantially more soluble in the extractant than said emulsifier; the process comprising the successive steps of:
a. combining the pumpable emulsion with the extractant under mixing conditions;
b. allowing the formation of granules containing the emulsifier and the pharmaceutically active substance;
c. collecting the granules and separating them from the extractant.

11. Process according to claim 10, wherein the pumpable emulsion and the extractant are combined by feeding a stream of the pumpable emulsion and a stream of the extractant into a mixing zone where both streams are thoroughly mixed.

12. Process according to claim 10 or 11, wherein the emulsion and the extractant arc admixed in a weight ratio within the range of 1:10 to 1:300.

13. Process according to any one of claims 10-12, wherein the organic polar solvent is selected from the group consisting of: C₁₋₆ alchohols, ketones, water and combinations thereof.

14. Process according to any one of claims 10-13, wherein the extractant is selected from the group consisting of carbon dioxide, nitrous oxide, ethane, ethylene propane, cyclopropane, propylene, butane, argon, nitrogen and mixtures thereof.

## Patentansprüche

1. Granulat, das einen volumengewichteten mittleren Durchmesser von 1-200 µm hat und enthält:
• mindestens 0,1 Gew.-% eines pharmazeutisch aktiven Wirkstoffes;
• mindestens 10 Gew.-% Emulgator, ausgewählt aus der Gruppe, bestehend aus Zuckerfettsäureestem, Monoglyceriden, Diglyceriden, Diacetylweinsäurester von Monoglycerid, Diacetylweinsäureester von Diglycerid, Polyglycerinester, Kalziumstearoyllactylat, Natriumstearoyllactylat und Kombinationen von diesen; und
• 0-89,9 Gew.-% eines wasserdispergierbaren Saccharids;
• wobei die Kombination des pharmazeutisch aktiven Wirkstoffs, des Emulgators und des wasserdispergierbaren Sacharids zusammen mindestens 60 Gew.-% des Granulats darstellen;
wobei das Granulat einphasig ist oder wobei das Granulat eine dispergierte Phase enthält,
die den pharmazeutisch aktiven Wirkstoff enthält, wobei die dispergierte Phase einen volumengewichteten mittleren Durchmesser von weniger als 300 nm hat.

2. Granulat nach Anspruch 1, wobei der Emulgator einen HLB-Wert von mehr als 6, vorzugsweise von 8-18 hat.

3. Granulat nach Anspruch 1 oder 2, wobei der Emulgator ein Zuckerfettsäureester ist.

4. Granulat nach einem der vorangehenden Ansprüche, wobei der pharmazeutisch aktive Wirkstoff ausgewählt ist aus der Gruppe bestehend aus Cannabinoiden, Steroiden, Flavonoiden, Polyphenolen und Kombinationen von diesen.

5. Granulat nach einem der vorangehenden Ansprüche, wobei das wasserdispergierbare Saccharid ein Molekulargewicht von weniger als 10.000, vorzugsweise von weniger als 6.000 g hat.

6. Feste oder halbfeste pharmazeutische Dosiereinheit, enthaltend mindestens 10 Gew.%, vorzugsweise mindestens 20 Gew.-% des Granulats gemäß einem der vorangehenden Ansprüche.

7. Verwendung des Granulats nach einem der Ansprüche 1 bis 5 für die Herstellung einer pharmazeutischen Dosiereinheit.

8. Verwendung nach Anspruch 7, wobei die pharmazeutische Dosiereinheit für die Verwendung in einem Verfahren zur therapeutischen oder prophylaktischen Behandlung vorgegeben ist, wobei die Behandlung eine transmukosale Verabreichung der pharmazeutischen Dosiereinheit enthält.

9. Verwendung gemäß Anspruch 8, wobei die Behandlung sublinguale, bukkale, pulmonare oder perorale Anwendung der pharmazeutischen Dosiereinheit enthält.

10. Verfahren zur Herstellung eines Granulats, enthaltend einen pharmazeutisch aktiven Wirkstoff, wobei das Verfahren anwendet:
• eine pumpbare Emulsion, enthaltend (i) eine kontinuierliche Phase, enthaltend mindestens 30 Gew.-% eines polaren Lösungsmittels und (ii) eine dispergierte Phase, enthaltend mindestens 10 Gew.-% eines Emulgators und mindestens 0,1 Gew.-% eines pharmazeutisch aktiven Wirkstoffs;
• ein Extraktionsmittel, enthaltend mindestens 60 Gew.-% eines überkritischen, unterkritischen oder verflüssigten Gases;
wobei das Lösungsmittel substantiell mehr in dem Extraktionsmittel löslich ist als der
Emulgator; wobei das Verfahren die zusätzlichen Schritte enthält:
a. Zusammenbringen der pumpfähigen Emulsion mit dem Lösungsmittel unter Mischbedingungen;
b. Zulassen der Bildung von Granulat, enthaltend den Emulgator und den pharmazeutisch aktiven Wirkstoff;
c. Sammeln des Granulats und Abtrennen von dem Lösungsmittel.

11. Verfahren nach Anspruch 10, wobei die pumpfähige Emulsion und das Extraktionsmittel durch Zuführen eines Stroms der pumpbaren Emulsion und eines Stroms des Extraktionsmittels in eine Mischzone zusammengeführt werden, wo beide Ströme gründlich gemischt werden.

12. Verfahren nach Anspruch 10 oder 11, wobei die Emulsion und das Extraktionsmittel in einem Gewichtsverhältnis innerhalb des Bereichs von 1:10 bis 1:300 zusammengemischt werden.

13. Verfahren nach einem der Ansprüche 10-12, wobei das organische polare Lösungsmittel aus der Gruppe ausgewählt ist, bestehend aus: C₁₋₆-Alkoholen, Ketonen, Wasser und Kombinationen von diesen.

14. Verfahren nach einem der Ansprüche 10-13, wobei das Extraktionsmittel ausgewählt ist aus der Gruppe bestehend aus Kohlendioxid, Stickstoffoxid, Ethan, Ethylenpropan, Cyclopropan, Propylen, Butan, Argon, Stickstoff und Mischungen von diesen.

## Revendications

1. Granulat ayant un diamètre moyen pondéré en volume de 1 à 200 µm et contenant :
• au moins 0,1 % en poids d'une substance pharmaceutiquement active ;
• au moins 10 % en poids d'un émulsifiant choisi dans le groupe constitué par les esters d'acide gras de sucre, les mono-glycérides, les di-glycérides, un ester d'acide diacétyltartrique de monoglycéride, les esters d'acide diacétyltartrique de diglycéride, les esters de polyglycérol, le stéaroyl lactylate de calcium, le stéaroyl lactylate de sodium et leurs combinaisons ;
et
• 0 à 89,9 % en poids d'un saccharide dispersible dans l'eau ;
• la combinaison de la substance pharmaceutiquement active, de l'émulsifiant et du saccharide dispersible dans l'eau représentant ensemble au moins 60 % en poids du granulé ;
dans lequel le granulat est monophasique ou dans lequel le granulat comprend une phase dispersée contenant la substance pharmaceutiquement active, ladite phase dispersée ayant un diamètre moyen pondéré en volume inférieur à 300 nm.

2. Granulat selon la revendication 1, dans lequel l'émulsifiant a une valeur HLB supérieure à 6, de préférence de 8 à 18.

3. Granulat selon la revendication 1 ou 2, dans lequel l'émulsifiant est un ester d'un acide gras et d'un sucre.

4. Granulat selon l'une quelconque des revendications précédentes, dans lequel la substance pharmaceutiquement active est choisie dans le groupe constitué par les cannabinoïdes, les stéroïdes, les flavonoïdes, les polyphénols et leurs combinaisons.

5. Granulat selon l'une quelconque des revendications précédentes, dans lequel le saccharide dispersible dans l'eau présente une masse moléculaire inférieure à 10 000, de préférence inférieure à 6 000 g.

6. Unité posologique pharmaceutique solide ou semi-solide comprenant au moins 10 % en poids, de préférence au moins 20 % en poids d'un granulat selon l'une quelconque des revendications précédentes.

7. Utilisation d'un granulat selon l'une quelconque des revendications 1 à 5 dans la fabrication d'une unité posologique pharmaceutique.

8. Utilisation selon la revendication 7, dans laquelle l'unité posologique pharmaceutique est destinée à une utilisation dans un procédé de traitement thérapeutique ou prophylactique, ledit traitement comprenant la administration transmucosique de l'unité posologique pharmaceutique.

9. Utilisation selon la revendication 8, dans laquelle le traitement comprend l'administration sublinguale, buccale, pulmonaire ou pérorale de l'unité posologique pharmaceutique.

10. Procédé de préparation d'un granulat contenant une substance pharmaceutiquement active, le procédé employant :
• une émulsion pompable comprenant (i) une phase continue contenant au moins 30 % en poids d'un solvant polaire et (ii) une phase dispersée contenant au moins 10 % en poids d'un émulsifiant et au moins 0,1 % en poids d'une substance pharmaceutiquement active ;
• un extractant comprenant au moins 60 % en poids d'un gaz liquéfié supercritique ou subcritique ;
ledit solvant étant sensiblement plus soluble dans l'extractant que ledit émulsifiant ; le procédé comprenant les étapes successives consistant à :
a. combiner l'émulsion pompable avec l'extractant dans des conditions de mélange ;
b. permettre la formation de granules contenant l'émulsifiant et la substance pharmaceutiquement active ;
c. collecter les granules et les séparer de l'extractant.

11. Procédé selon la revendication 10, dans lequel l'émulsion pompable et l'extractant sont combinés par alimentation d'une zone de mélange avec un courant de l'émulsion pompable et un courant du solvant de l'extractant, zone de mélange où les deux courants sont mélangés méticuleusement.

12. Procédé selon la revendication 10 ou 11, dans lequel l'émulsion et l'extractant sont mélangés dans un rapport en poids dans la plage de 1 : 10 à 1 : 300.

13. Procédé selon l'une quelconque des revendications 10 à 12, dans lequel le solvant polaire organique est choisi dans le groupe constitué par : les alcools en C₁ à 6, les cétones, l'eau et leurs combinaisons.

14. Procédé selon l'une quelconque des revendications 10 à 13, dans lequel l'extractant est choisi dans le groupe constitué par le dioxyde de carbone, l'oxyde nitreux, l'éthane, l'éthylène, le propane, le cyclopropane, le propylène, le butane, l'argon, l'azote et leurs mélanges.
